# EUROPEAN PATENT APPLICATION

(11) **EP 3 399 023 A1**
(43) Date of publication of application: **07.11.2018**
(21) Application number: 16881681.7
(22) Date of filing: 21.12.2016
(51) Int. Cl.: C12N 5/071, C12N 5/074, C12N 5/0775, C12M 1/00, C12M 1/22

(54) **METHOD FOR PROMOTING DIFFERENTIATION OF CULTURED CELLS AND CULTURED CELL DIFFERENTIATION-PROMOTING AGENT**

(30) Priority: 28.12.2015 JP 2015256336
(71) Applicant: Zeon Corporation, Tokyo 100-8246 (JP)
(72) Inventor: ICHIMURA, Naoya, Tokyo 100-8246 (JP)
(74) Representative: Maiwald Patent- und Rechtsanwaltsgesellschaft mbH
(86) International application number: PCT/JP2016/088207
(87) International publication number: WO 2017/115706

(57) **Abstract**

The invention is a method for promoting differentiation of cultured cells, including bringing a norbornene-based addition polymer formed article into contact with cells being cultured, and a cultured cell differentiation-promoting agent including a norbornene-based addition polymer formed article. The invention provides a method that can further promote the differentiation of cultured cells, and a cultured cell differentiation-promoting agent that may suitably be used for the method.

## Description

### TECHNICAL FIELD

The present invention relates to a method for promoting the differentiation of cultured cells, and a cultured cell differentiation-promoting agent.

### BACKGROUND ART

In recent years, regenerative medicine that utilizes pluripotent cells and stem cells (e.g., ES cells and iPS cells) and mesenchymal stem cells has attracted attention. Pluripotent cells and stem cells are said to be undifferentiated cells that have not differentiated. According to regenerative medicine, these undifferentiated cells are caused to differentiate into the desired internal organ, tissue, or cells, and the patient is treated using the resulting internal organ, tissue, or cells. For example, adipose-derived stem cells sampled from subcutaneous adipose tissues of a patient can be differentiated into adipocytes through preadipocytes, or differentiated into osteocytes or chondrocytes by differentiation induction culture. Orthopaedic surgical treatment and aesthetic treatment that utilize such stem cells have been implemented. In addition, basal cells/squamous cells on epidermis are taken from a skin and subjected to differentiation induction culture, so that they are differentiated into granular cells/horny cells to prepare epidermal sheets, which are applied for treatment. For example, adipose-derived stem cells contained in adipose tissues recovered by subcutaneous adipose suction as aesthetic surgery are subjected to differentiation induction culture, which are used for treating obesity (Patent Document 1), and for treating bones (Patent Document 2).

As described above, utilization and practical use of stem cells have been promoted, but it takes as long as several weeks for differentiation culture of the stem cells, which is a problem for practical use.

In addition, a dish, a flask, or the like made of polystyrene that has been subjected to a hydrophilization treatment, has been widely used as a culture vessel when performing the differentiation-inducing operation.

### CITATION LIST

### PATENT LITERATURE

PTL 1: Japanese Unexamined Patent Application Publication No. 2014-520531 (WO 2013/003595 A1)
PTL 2: Japanese Unexamined Patent Application Publication No. 2012-525377 (US 20120020937 A1)

### SUMMARY OF INVENTION

### TECHNICAL PROBLEM

The invention has been made in view of the above situation. An object of the present invention is to provide a method that can promote the differentiation of cultured cells, and a cultured cell differentiation-promoting agent.

### SOLUTION TO PROBLEM

The inventor conducted extensive studies to solve the above problem. As a result, the inventor have found that the amount of mRNA (i.e., differentiation marker) increases when the differentiation target cells are cultured while bringing the differentiation target cells into contact with a norbornene-based addition polymer. This finding has led to the completion of the invention.

Several aspects of the invention provide the following method for promoting the differentiation of cultured cells (see (1) and (2)) and a cultured cell differentiation-promoting agent (see (3)).
(1) A method for promoting differentiation of cultured cells including bringing a norbornene-based addition polymer formed article into contact with cells that are being cultured.
(2) The method for promoting differentiation of cultured cells according to (1), wherein the cells that are being cultured are stem cells.
(3) A cultured cell differentiation-promoting agent including a norbornene-based addition polymer formed article.

### ADVANTAGEOUS EFFECTS OF INVENTION

The invention thus provides a method that can further promote the differentiation of cultured cells, and a cultured cell differentiation-promoting agent that may suitably be used for the method.

### BRIEF DESCRIPTION OF DRAWINGS

FIG. 1 is a graph illustrating a PPAPγ expression level.
FIG. 2 is an electron micrograph illustrating differentiated cells cultured in the dish according to the present invention.
FIG. 3 is an electron micrograph illustrating cells cultured in a dish in Comparative Example.

### DESCRIPTION OF EMBODIMENTS

The present invention relates to a method for promoting differentiation of cultured cells, including bringing a norbornene-based addition polymer formed article into contact with cells being cultured.

The cells used in connection with the invention are not particularly limited as long as the cells can differentiate. Examples of the cells that can differentiate include embryonic stem cells; differentiated pluripotent stem cells; various stem cells including mesenchymal stem cells such as myelic mesenchymal stem cells and adipose-derived stem cells; various precursor cells; cells that have differentiated from an ectoderm, a mesoderm, or an endoderm, and have not reached a terminal differentiation state; and the like. Above all, adipose-derived stem cells obtained from subcutaneous adipose tissue can be mentioned as preferable examples. The adipose-derived stem cells can be obtained based on adipose tissue collected from subcutaneous adipose by centrifugation or the like.

A liquid medium is normally used when culturing the cells.

A liquid medium is normally used that has a pH buffer action, has an osmotic pressure suitable for the cells, includes nutritional ingredients for the cells, and does not have toxicity to the cells.

Examples of a component that provides a pH buffer action to the liquid medium include Tris hydrochloride, a phosphate, a carbonate, and the like.

The osmotic pressure of the liquid medium is normally adjusted using an aqueous solution that includes potassium ions, sodium ions, calcium ions, glucose, and the like at an adjusted concentration so that the osmotic pressure of the liquid medium is almost equal to that of the cells.

Examples of such an aqueous solution include physiological saline such as phosphate buffered saline, Tris-buffered saline, and HEPES-buffered saline; a Ringer's solution such as Ringer's lactate solution, Ringer's acetate solution, and Ringer's bicarbonate solution; and the like.

Examples of the nutritional ingredients for the cells include an amino acid, a nucleic acid, a vitamin, a mineral, and the like.

A commercially-available product such as RPMI-1640, HAM, α-MEM, DMEM, EMEM, F-12, F-10, and M-199 may be used as the liquid medium.

An additive may be added to the liquid medium. Examples of the additive include an inducer such as a protein, a low-molecular-weight compound having differentiation-inducing activity, a mineral, a metal, a vitamin component, and the like. It is preferable to add an additive that induces differentiation to the liquid medium.

Examples of the additive that induces differentiation include a ligand, an agonist, and an antagonist that act on a cell surface acceptor; a ligand, an agonist, and an antagonist that act on a nuclear receptor; an extracellular matrix such as collagen and fibronectin; part of the extracellular matrix, or a compound that imitates the extracellular matrix; a component that acts on a protein that is involved in a cell signaling pathway; a component that acts on a primary or secondary metabolism enzyme within the cells; a component that affects intranuclear or intramitochondrial gene expression; DNA and RNA that can be introduced into the cells in combination with a virus vector or the like; and the like.

These additives may be used either alone or in combination.

Naturally, various commercial differentiation mediums for stem cells containing these additives may also be used.

The cell culture conditions are not particularly limited. The cell culture conditions may be appropriately determined taking account of the cells and the object. For example, the cells may be cultured using a humidified incubator that contains carbon dioxide at a concentration of about 5%, and is maintained at a constant temperature within the range from 20°C to 37°C.

The norbornene-based addition polymer formed article used in connection with the invention is obtained by forming a norbornene-based addition polymer so as to have an arbitrary shape.

The norbornene-based addition polymer is obtained by addition polymerization of a norbornene-based monomer, and specifically includes an addition polymer of norbornene-based monomers, and an addition polymer of norbornene-based monomers and additional monomers copolymerizable therewith, and the like.

Examples of the norbornene-based monomer include a bicyclic norbornene-based monomer such as bicyclo[2.2.1]hept-2-ene (trivial name: norbornene), 5-methylbicyclo[2.2.1]hept-2-ene, 5,5-dimethylbicyclo[2.2.1]hept-2-ene, 5-ethylbicyclo[2.2.1]hept-2-ene, 5-ethylidenebicyclo[2.2.1]hept-2-ene, 5-vinylbicyclo[2.2.1]hept-2-ene, 5-propenylbicyclo[2.2.1]hept-2-ene, 5-methoxycarbonylbicyclo[2.2.1]hept-2-ene, 5-cyanobicyclo[2.2.1]hept-2-ene, and 5-methyl-5-methoxycarbonylbicyclo[2.2.1]hept-2-ene; a tricyclic norbornene-based monomer such as tricyclo[4.3.0^{1,6}.1^{2,5}]deca-3,7-diene (trivial name: dicyclopentadiene), 2-methyldicyclopentadiene, 2,3-dimethyldicyclopentadiene, and 2,3-dihydroxydicyclopentadiene; a tetracyclic norbornene-based monomer such as tetracyclo[4.4.0.1^{2,5}.1^{7,10}]-3-dodecene(tetracyclododecene), tetracyclo[4.4.0.1^{2,5}.1^{7,10}]-3-dodecene,8-methyltetracyclo[4.4.0.1^{2,5}.1^{7,10}]-3-dodecene, 8-ethyltetracyclo[4.4.0.1^{2,5}.1^{7,10}]-3-dodecene, 8-ethylidenetetracyclo[4.4.0.1^{2,5}.1^{7,10}]-3-dodecene, 8,9-dimethyltetracyclo[4.4.0.1^{2,5}.1^{7,10}]-3-dodecene, 8-ethyl-9-methyltetracyclo[4.4.0.1^{2,5}.1^{7,10}]-3-dodecene, 8-ethylidene-9-methyltetracyclo[4.4.0.1^{2,5}.1^{7,10}]-3-dodecene, 8-methyl-8-carboxymethyltetracyclo[4.4.0.1^{2,5}.1^{7,10}]-3-dodecene, 7,8-benzotricyclo[4.3.0.1^{2,5}]dec-3-ene (trivial name: methanotetrahydrofluorene (also referred to as 1,4-methano-1,4,4a,9a-tetrahydrofluorene)), 1,4-methano-8-methyl-1,4,4a,9a-tetrahydrofluorene, 1,4-methano-8-chloro-1,4,4a,9a-tetrahydrofluorene, and 1,4-methano-8-bromo-1,4,4a,9a-tetrahydrofluorene; and the like.

Examples of an additional monomer that can undergo addition copolymerization with the norbornene-based monomer include an α-olefin-based monomer having 2 to 20 carbon atoms, such as ethylene, propylene, 1-butene, 1-pentene, and 1-hexene; a cycloolefin-based monomer such as cyclobutene, cyclopentene, cyclohexene, cyclooctene, and tetracyclo[9.2.1.0^{2,10}.0^{3,8}]tetradeca-3,5,7,12-tetraene (also referred to as 3a,5,6,7a-tetrahydro-4,7-methano-1H-indene); a non-conjugated diene-based monomer such as 1,4-hexadiene, 4-methyl-1,4-hexadiene, 5-methyl-1,4-hexadiene, and 1,7-octadiene; and the like. Among these, an α-olefin-based monomer is preferable, and ethylene is more preferable.

These monomers may be substituted with one substituent, or may be substituted with two or more substituents. Examples of the substituent include an alkyl group, an alkylene group, an aryl group, a silyl group, an alkoxycarbonyl group, an alkylidene group, and the like.

An addition polymer of a norbornene-based monomer, or an addition polymer of a norbornene-based monomer and an additional monomer that can undergo addition copolymerization with the norbornene-based monomer, may be obtained by polymerizing the monomer component in the presence of a known addition polymerization catalyst.

Examples of the addition polymerization catalyst include a conventionally-known addition polymerization catalyst that includes a titanium, zirconium, or vanadium compound and an organoaluminum compound.

In the norbornene-based addition polymer used in connection with the invention, the limiting viscosity [η] measured in decalin at 135°C is normally 0.01 to 20 dl/g, preferably 0.03 to 10 dl/g, and more preferably 0.05 to 5 dl/g from the viewpoint of the strength of the formed article, and the melt flow rate (MFR) measured at 260°C under a load of 2.16 kg in accordance with ASTM D1238 is normally 0.2 to 200 g/10 min, preferably 1 to 100 g/10 min, and more preferably 5 to 50 g/10 min from the viewpoint of formability.

The glass transition temperature of the norbornene-based addition polymer may be appropriately selected taking account of the intended use. The glass transition temperature of the norbornene-based addition polymer is normally 50 to 300°C, preferably 70 to 280°C, particularly preferably 70 to 250°C, and still more preferably 80 to 200°C. When the glass transition temperature of the norbornene-based addition polymer is within the above range, the norbornene-based addition polymer exhibits heat resistance and formability in a highly balanced manner, which is suitable.

Note that the glass transition temperature of the norbornene-based addition polymer refers to a value measured in accordance with JIS K 7121.

The softening point of the norbornene-based addition polymer refers to a softening point measured by a thermal mechanical analyzer (TMA), and is normally 30°C or higher, preferably 70°C or higher, and more preferably 80 to 260°C from the viewpoint of formability. The crystallinity of the norbornene-based addition polymer measured by X-ray diffractometry is normally 20% or lower, preferably 10% or lower, and more preferably 2% or lower from the viewpoints of transparency and dimensional controllability of the formed article.

The norbornene-based addition polymers described above may be used either alone or in combination.

An additive that is normally used for a thermoplastic resin material, such as a soft polymer, an antioxidant, a UV absorber, a light stabilizer, a near-infrared absorber, a release agent, a coloring agent (e.g., dye and pigment), a plasticizer, an antistatic agent, and a fluorescent whitening agent, may be added to the norbornene-based addition polymer in an amount that is normally employed.

In addition, an additional polymer other than the soft polymer (hereinafter referred to as "additional polymer") may be mixed with the norbornene-based addition polymer. The additional polymer is normally mixed with the norbornene-based addition polymer in a ratio of 200 parts by weight or less, preferably 150 parts by weight or less, and more preferably 100 parts by weight or less, based on 100 parts by weight of the norbornene-based addition polymer.

If the ratio of the additive or the additional polymer is added too large based on the norbornene-based addition polymer, the effect of promoting the differentiation of cells may decrease. Therefore, it is preferable to add (mix) the additive and the additional polymer within such a range that the properties of the norbornene-based addition polymer are not impaired.

The additive and the additional polymer may be mixed with the norbornene-based addition polymer using an arbitrary method as long as the additive and the additional polymer are sufficiently dispersed in the norbornene-based addition polymer. The additive and the additional polymer may be added in an arbitrary order. Examples of the mixing method include a method that mixes (kneads) the resin in a molten state using a mixer, a single-screw kneader, a twin-screw kneader, a roll, a Brabender, an extruder, or the like, a method that dissolves the resin in an appropriate solvent to effect dispersion, and removes the solvent using a coagulation method, a casting method, or a direct drying method, and the like.

When the resin is mixed (kneaded) using a twin-screw kneader, the resulting mixture (kneaded product) is normally extruded in the shape of a rod in a molten state, and cut (pelletized) to have an appropriate length using a strand cutter.

A forming method used when forming the norbornene-based addition polymer may be arbitrarily selected taking account of the shape of the norbornene-based addition polymer formed article that is brought into contact with cells. Examples of the forming method include an injection forming method, an extrusion method, a cast forming method, an inflation forming method, a blow forming method, a vacuum forming method, a press forming method, a compression forming method, a rotational forming method, a calendering method, a roll forming method, a cutting method, a spinning method, and the like. Note that these methods may be used in combination, and a post-treatment such as stretching may optionally be performed after forming.

The resulting formed article is used as the cultured cell differentiation-promoting agent according to the invention.

The norbornene-based addition polymer formed article used in connection with the invention may be any formed article in which at least a surface in contact with the cells is made of the norbornene-based addition polymer formed article, and the whole formed article does not have to be made of an alicyclic structure-containing polymer.

The shape of the norbornene-based addition polymer formed article is not particularly limited. The norbornene-based addition polymer formed article may have a plate-like shape, a powdery shape, a granular shape, a string-like shape, a sheet-like shape, or any other shape. The surface of the norbornene-based addition polymer formed article may be flat or irregular. The norbornene-based addition polymer formed article may be a hollow formed article. A plurality of formed articles that differ in shape may be combined through an adhesive or the like, or may be combined without using an adhesive or the like, to form another formed article.

The norbornene-based addition polymer formed article may be a member that forms part or the entirety of a culture vessel (e.g., dish, plate, bag, tube, scaffold, cup, and jar fermenter), a part of a culture apparatus (e.g., stirring blade, stirring bar, baffle, and connection tube), a culture tool used for culture operation (e.g., pipette, stirring device, filter, and cell scraper), and the like, as long as the norbornene-based addition polymer formed article can be brought into contact with the cells.

When implementing the method according to the invention, there is a tendency that the cells that are being cultured survive in the culture medium in a suspended state irrespective of whether the cells are adherent cells or suspension cells. Therefore, the norbornene-based addition polymer formed article can be brought into contact with the cells in various ways.

When implementing the invention, it is preferable to sterilize the formed article before bringing the formed article into contact with the cultured cells. The sterilization method is not particularly limited. The sterilization method may be selected from sterilization methods that are normally employed in the medical field (e.g., a heating method such as a high-pressure steam method and a dry heat method; a radiation method that applies radiation such as y-rays or an electron beam, and an irradiation method that applies high-frequency waves; a gas method that brings a gas such as ethylene oxide gas (EOG) into contact with the sterilization target; and a filtration method that utilizes a sterilization filter) taking account of the shape of the formed article and the cells to be cultured.

The surface of the formed article may also be subjected to a treatment (e.g., plasma treatment, corona discharge treatment, ozone treatment, and UV irradiation treatment) other than a sterilization treatment that is normally applied to a culture vessel.

The norbornene-based addition polymer formed article (i.e., the cultured cell differentiation-promoting agent according to the invention) may be brought into contact with the cultured cells using an arbitrary method taking account of the shape of the cultured cell differentiation-promoting agent. For example, the norbornene-based addition polymer formed article may be brought into contact with the cultured cells using a method that cultures the cells in a culture medium into which the norbornene-based addition polymer formed article (cultured cell differentiation-promoting agent) is mixed; a method that cultures the cells in a culture vessel that is formed using the norbornene-based addition polymer; a method that cultures the cells using a culture tool that is formed using the norbornene-based addition polymer; or the like. These methods may be used in combination.

Since cells have a signaling capability, all of the cultured cells need not necessarily come in contact with the norbornene-based addition polymer formed article, and the cultured cells need not necessarily come in contact with the norbornene-based addition polymer formed article during the entire culture period. Note that it is preferable that the contact time be as long as possible to compensate for a decrease in effect with the passing of time.

The temperature at which the norbornene-based addition polymer formed article is brought into contact with the cultured cells is not particularly limited as long as the cells can be grown.

### EXAMPLES

The invention is further described below by way of examples. Note that the invention is not limited to the following examples.

### [Production Example 1]

A culture dish having a bottom diameter of 3 cm was formed using "APEL (registered trademark) APL6013T" manufactured by Mitsui Chemicals, Inc. and "TOPAS (registered trademark) 6013" manufactured by Polyplastics Co., Ltd. as norbornene-based addition polymers (hereinafter, referred to as "APEL dish" and "TOPAS dish" respectively) by injection forming, and then the TOPAS dish and the APEL dish were sterilized by a gas method using EOG (ethylene oxide gas).

### [Example 1]

Using a culture medium for adipose-derived stem cells (pH 7.4) containing DMEM/Ham's F-12 (1:1, v/v), a buffer (HEPES; 4-(2-hydroxyethyl)-1-piperazineethanesulfonic acid), fetal bovine serum, penicillin, streptomycin and amphotericin B, an adipose-derived stem cell (manufactured by ZenBio, Inc.) was respectively seeded onto the TOPAS dish and the APEL dish at a cell density of 1×10⁴ cells/cm², and cultured in a CO₂ incubator for 10 days. Hereinafter, the samples cultured in the TOPAS dish and the APEL dish are referred to as "TOPAS dish-precultured sample" and "APEL dish-precultured sample" respectively.

### [Comparative Example 1]

Cells were cultured in the same manner as in Example 1 for 10 days, except that a cell culture dish manufactured by Corning Incorporated [Falcon (registered trademark) 353001] (hereinafter referred to as "polystyrene dish") were used instead of the TOPAS dish or the APEL dish in Example 1. The resulting sample is hereinafter referred to as "polystyrene dish-cultured sample".

### [Comparative Example 2]

Using the same medium as in Comparative Example 1, an adipose-derived stem cell (manufactured by ZenBio, Inc.) was seeded onto the polystyrene dish at a cell density of 1×10⁴ cells/cm², cultured in the CO₂ incubator for 3 days, and then the whole medium was removed. Instead, a culture medium for adipocyte differentiation containing DMEM/Ham's F-12 (1:1, v/v), a buffer (HEPES), fetal bovine serum, penicillin, streptomycin, amphotericin B, biotin, pantothenate, human insulin, dexamethasone, isobutylmethylxanthine and PPAPγ agonist was added, and the cell was further cultured in the CO₂ incubator for 7 days. The resulting sample is hereinafter referred to as "polystyrene dish-differentiation-cultured sample".

### [Evaluation of differentiation state of cells]

In Example 1 and Comparative Example 1, a differentiation marker that was expressed in the cells, was analyzed as described below to evaluate the differentiation state of the cells. PPAPγ that is a regulator gene for adipocyte differentiation was used as an index with respect to the differentiation marker.

After completion of the culture period, cells were collected from each sample, and the amount of PPARy mRNA contained in the cells was quantitatively determined by Real-Time PCR (see below). The mRNA of GAPDH was used as an internal standard.

RNA was extracted from the cells using CellAmp Direct Prep Kit for RT-PCR (Real Time) (manufactured by Takara Bio Inc.), and a PCR reaction was carried out using the extracted sample as a template sample and CellAmp Direct RNA Prep Kit for One Step RT-PCR (Real Time) (manufactured by Takara Bio Inc.) as a Real Time PCR reaction reagent, in PCR-CFX96 (Bio-Rad Laboratories, Inc.) system.

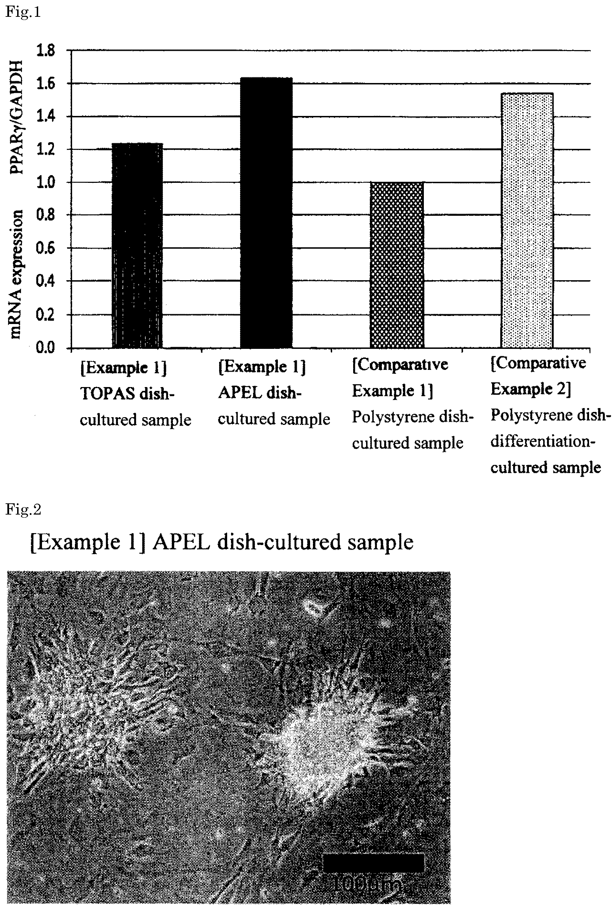

The measured results of the PPAPγ mRNA expression level (calibrated with GAPDH mRNA expression level) are shown in Figure 1. Figure 1 shows relative values of the PPAPγ expression levels in the TOPAS dish-cultured sample and the APEL dish-cultured sample in Example 1, and the PPAPγ expression level in the polystyrene dish-differentiation-cultured sample in Comparative Example 2, relative to the PPAPγ expression level in the polystyrene dish-cultured sample in Comparative Example 1.

The expression level of the PPAPγ as an adipocyte differentiation marker is increased in the TOPAS dish-cultured sample and the APEL dish cultured-sample compared to the expression level in the polystyrene culture vessel (Example 1, Comparative Example 1), and it can be seen that the differentiation marker in APEL dish is detected to the same extent as the case of using the differentiation medium in the polystyrene dish, without using the differentiation medium (Example 1, Comparative Example 2).

This shows that the cell differentiation is promoted by bringing the cells into contact with the norbornene-based addition polymer.

In addition, the APEL dish-cultured sample (day 10 of culture) in Example 1 was observed with a phase contrast microscope. The result is shown in Figure 2.

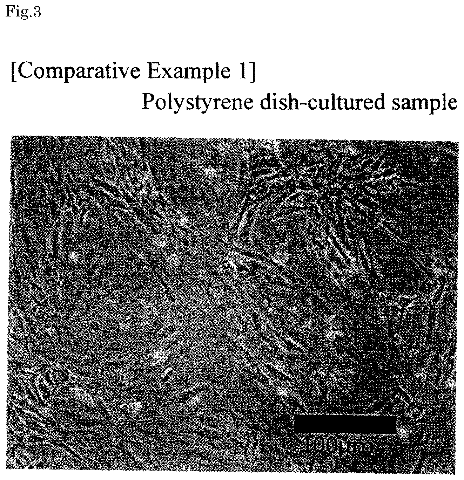

In addition, the polystyrene dish-cultured sample (day 10 of culture) in Comparative Example 1 was observed with a phase contrast microscope. The result is shown in Figure 3.

It is shown that cell aggregations are formed and also accumulation of oil droplets is enhanced in the APEL dish-cultured sample of Figure 2, compared to the polystyrene dish-cultured sample shown in Figure 3.

## Claims

1. A method for promoting differentiation of cultured cells, including bringing a norbornene-based addition polymer formed article into contact with cells being cultured.

2. The method for promoting differentiation of cultured cells according to claim 1, wherein the cells that are being cultured are stem cells.

3. The method for promoting differentiation of cultured cells according to claim 2, wherein the cells that are being cultured are adipose-derived stem cells.

4. A cultured cell differentiation-promoting agent including a norbornene-based addition polymer formed article.
